(19) Europäisches Patentamt
European Patent Office
Office européen des brevets

(11) EP 2 423 324 A1

(12) **EUROPEAN PATENT APPLICATION**
published in accordance with Art. 153(4) EPC

(43) Date of publication:
**29.02.2012 Bulletin 2012/09**

(21) Application number: **10767115.8**

(22) Date of filing: **22.04.2010**

(51) Int Cl.:
***C12Q 1/68*** *(2006.01)* ***C12N 15/09*** *(2006.01)*

(86) International application number:
**PCT/JP2010/057135**

(87) International publication number:
**WO 2010/123058 (28.10.2010 Gazette 2010/43)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA ME RS**

(30) Priority: **22.04.2009 JP 2009104468**

(71) Applicant: **Vertex Pharmaceuticals Incorporated**
**Cambridge, MA 02139 (US)**

(72) Inventors:
- **KISHI, Yasuhiro**
**Osaka-shi**
**Osaka 541-8505 (JP)**
- **KAMIYA, Naohiro**
**Osaka-shi**
**Osaka 541-8505 (JP)**

(74) Representative: **Burrichter, Arwed Andreas**
**COHAUSZ & FLORACK**
**Patent- und Rechtsanwälte**
**Partnerschaftsgesellschaft**
**Bleichstrasse 14**
**40211 Düsseldorf (DE)**

# (54) PROBE SET FOR IDENTIFICATION OF NUCLEOTIDE MUTATION, AND METHOD FOR IDENTIFICATION OF NUCLEOTIDE MUTATION

(57) The type of a nucleotide located in a mutation site on a target nucleic acid can be identified by carrying out a fluorescence real-time PCR using a probe set comprising a detection probe and a counter probe, wherein the detection probe comprises an oligonucleotide which comprises a nucleotide sequence containing the mutation site on the target nucleic acid and also containing a nucleotide of interest in the mutation site or a nucleotide sequence complementary to the aforementioned nucleotide sequence, has a fluorescent substance added to the 5'-terminal and a quenching substance added to the 3'-terminal, and has, introduced therein, such a modification that the melting temperature of the probe becomes 3°C or more higher than that of the counter probe, and wherein the counter probe comprises an oligonucleotide which comprises a nucleotide sequence containing a mutation site and also containing a nucleotide that is different from the nucleotide of interest in the mutation site or a nucleotide sequence complementary to the aforementioned nucleotide sequence.

(A) Template: A156V Only

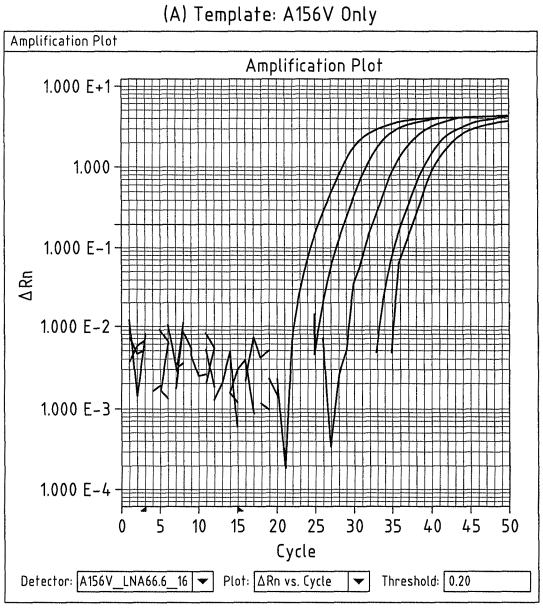


Fig.1A

EP 2 423 324 A1

(B) Template: A156V in $10^5$ wild

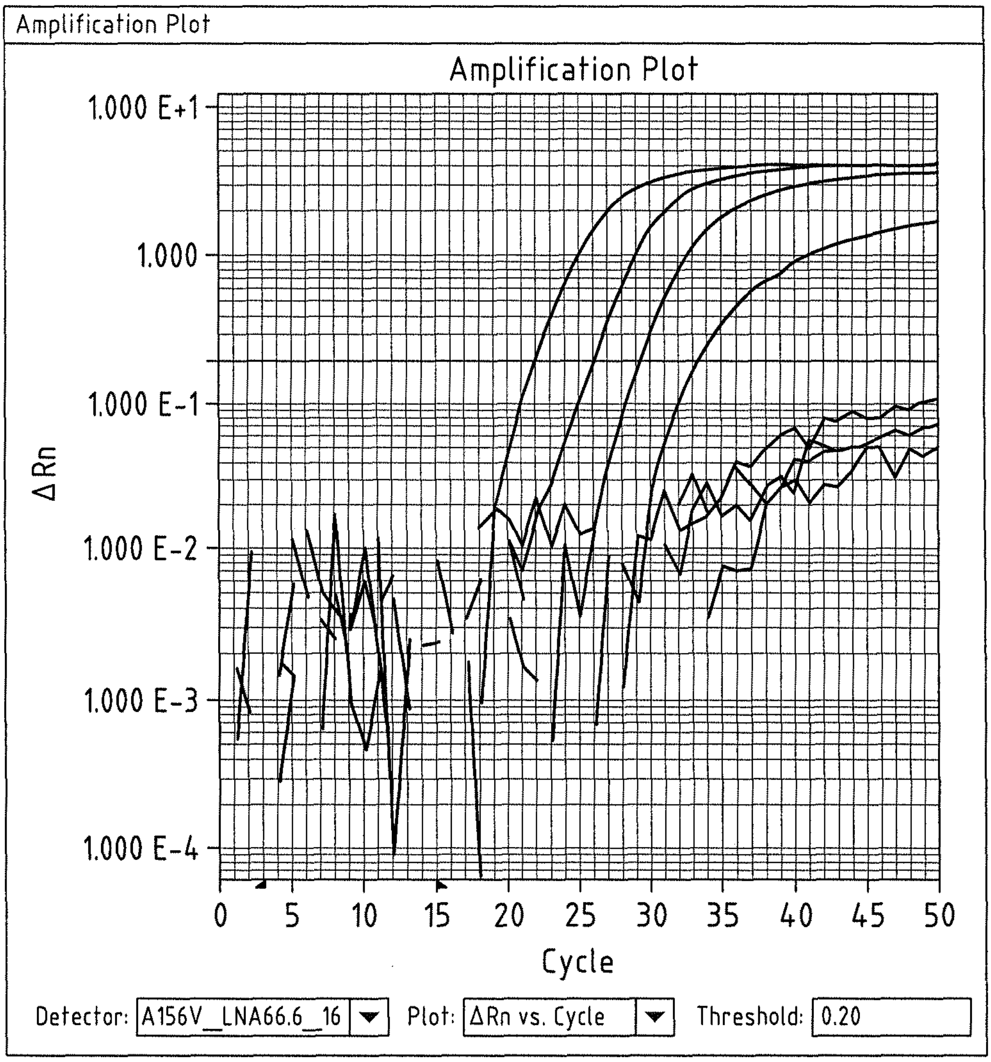


Fig.1B

## Description

TECHNICAL FIELD

The present invention relates to a probe set to be used for identifying the type of the nucleotide at a nucleotide mutation site, and a method for identifying a nucleotide mutation. The present invention also relates to a probe set for identifying a mutation involved in drug resistance of a virus and/or the like, and a method for identifying a mutation.

BACKGROUND ART

Nucleotide mutations (including nucleotide polymorphisms) are factors having large influences on phenotypes of organisms, and investigation of the type of a mutation is frequently carried out in order to predict its phenotype or to predict the effect of a drug. Examples of known methods for identifying the type of a mutated nucleotide include direct sequencing, the invader method, the method using a DNA chip on which polymorphism-specific probes are immobilized, and the allele-specific PCR method, but these are insufficient in view of the labor required for the identification and detection sensitivity, so that a method which allows simpler and more sensitive identification of the type of a mutated nucleotide has been demanded.
Further, although Non-patent Document 1 discloses a method for identifying mismatch of a nucleotide using a locked nucleic acid (LNA), it is a method based on detection by hybridization to a target sequence.
Hepatitis C virus (hereinafter also referred to as HCV) is an infectant which causes a human hepatic disorder, and it has been revealed that most of non-A non-B hepatitis in Japan is due to HCV. It has also been revealed that the lesion of an HCV-infected chronic hepatitis patient progresses to liver cirrhosis or hepatocellular carcinoma, and infection by blood transfusion has also been problematic.
As therapeutic agents for HCV, interferon, ribavirin and the like have been used, but, in recent years, protease inhibitors are being developed. However, depending on the type of the virus, existence of a mutant-type virus for which a protease inhibitor is not effective has been reported, and hence it is important, for effective therapy, to preliminarily detect the type of mutation and determine the medication policy based on its result.
However, it is difficult for conventional test methods to detect a virus with a low copy number, so that a method which allows accurate and highly sensitive detection has been demanded.
As a method to detect a protease inhibitor-resistant mutation of HCV, the TaqMan Mismatch Amplification Mutation Assay method has been reported (Non-patent Document 2). In this method, a mismatch is introduced to a primer, and an amplification signal is detected only in cases where a desired mutated sequence exists, Thus, in cases where a negative result is obtained, identification of the mutation requires another assay, the method is insufficient.

PRIOR ART DOCUMENTS

Non-patent Documents
Non-patent Document 1: Nucleic Acid Research, 2006, Vol. 34, No. 8, e60
Non-patent Document 2: Journal of Virological Methods, 2008, Vol, 153, p156-162

SUMMARY OF THE INVENTION

The present invention aims to provide a method by which a nucleotide mutation can be more simply and highly sensitively identified, and a reagent used therefor. The present invention also aims to provide a method for identifying a mutation in a virus genome involved in drug resistance or the like, and a reagent to be used therefor, more particularly, to provide a method for determining a nucleotide mutation in the NS3 protease gene in the HCV-1b type virus, which nucleotide mutation is useful for prediction of responsiveness of an individual to a protease inhibitor, and a reagent to be used therefor.
The present inventors intensively studied to solve the above problems. As a result, the present inventors discovered that a mutation of interest can be identified simply and highly sensitively by carrying out real-time PCR using a probe set comprising: a detection probe composed of an oligonucleotide having: a nucleotide sequence comprising said mutation site in said target nucleic acid, said mutation site comprising a nucleotide of interest; or a nucleotide sequence complementary to said nucleotide sequence; said oligonucleotide having a fluorescent substance attached to the 5'-end and a quencher attached to the 3'-end; and a counter probe composed of an oligonucleotide having: a nucleotide sequence comprising said mutation site, said mutation site comprising a nucleotide different from said nucleotide of interest; or a nucleotide sequence complementary to said nucleotide sequence, and wherein said detection

probe has a modification introduced such that the melting temperature thereof is not less than 3°C higher than the melting temperature of the counter probe.
Further, the present inventors discovered that, by designing the probe set for identification of a mutant-type virus, a virus having a mutation such as a drug resistance mutation can be identified and, thus, determination of the administration policy of an antiviral drug is possible, thereby completed the present invention.

(1) A probe set to be used for identifying the type of the nucleotide at a mutation site of a target nucleic acid by fluorescent real-time PCR, said probe set comprising:

a detection probe composed of an oligonucleotide having: a nucleotide sequence comprising said mutation site in said target nucleic acid, said mutation site comprising a nucleotide of interest; or a nucleotide sequence complementary to said nucleotide sequence; said oligonucleotide having a fluorescent substance attached to the 5'-end and a quencher attached to the 3'-end; and
a counter probe composed of an oligonucleotide having: a nucleotide sequence comprising said mutation site, said mutation site comprising a nucleotide different from said nucleotide of interest; or a nucleotide sequence complementary to said nucleotide sequence, and
wherein said detection probe has a modification introduced such that the melting temperature thereof is not less than 3 °C higher than the melting temperature of the counter probe.

(2) The probe set according to (1), wherein said modification is modification by a locked nucleic acid.
(3) The probe set according to (1) or (2), wherein said target nucleic acid is derived from a virus.
(4) The probe set according to (3), wherein said virus is hepatitis C virus.
(5) The probe set according to (4), wherein said hepatitis C virus belongs to type 1 b.
(6) The probe set according to (5), wherein said mutation is involved in drug resistance of said virus.
(7) The probe set according to (6), wherein said drug is a protease inhibitor.
(8) The probe set according to (6), wherein said drug is Telaprevir.
(9) The probe set according to (7) or (8), wherein said protease is NS3 protease of hepatitis C virus.
(10) The probe set according to (9), wherein said mutation is a mutation which results in replacement of any of the following amino acids in the wild-type NS3 protease:

(i) Ala at position 156;
(ii) Arg at position 155;
(iii) Ala at position 156 and Val at position 158;
(iv) Thr at position 54; and
(v) Val or Ile at position 132.

(11) A method for identifying the type of the nucleotide at a mutation site of a target nucleic acid, comprising carrying out fluorescent real-time PCR by using the probe set according to any one of (1) to (10).
(12) A method for predicting responsiveness of a hepatitis C patient to a protease inhibitor, comprising carrying out fluorescent real-time PCR by using the probe set according to any one of (7) to (10) to specify the type of the nucleotide at a mutation site involved in protease inhibitor resistance of hepatitis C virus type 1b and thereby determining whether or not said virus is a protease inhibitor-resistant virus.
(13) A method for treating hepatitis C, comprising predicting the responsiveness of a hepatitis C patient to a protease inhibitor by the method according to (12), and said protease inhibitor is administered in cases where a protease inhibitor resistance mutation is not detected in said hepatitis C patient, while said protease inhibitor is not administered or administration of said protease inhibitor is ceased in cases where a protease inhibitor resistance mutation is detected in said hepatitis C patient.
(14) A diagnostic kit for predicting responsiveness of a hepatitis C patient to a protease inhibitor, said kit comprising the probe set according to any one of (7) to (10).
(15) The diagnostic kit according to (14), said diagnostic kit comprising an instruction wherein a therapeutic guideline is described, which therapeutic guideline explains that (i) a protease inhibitor may be administered in cases where a protease inhibitor resistance mutation is not detected; and (ii) administration of a protease inhibitor is ceased or not carried out in cases where a protease inhibitor resistance mutation is detected.
(16) Use of the probe set according to any one of (7) to (10) for prediction of responsiveness of a hepatitis C patient to a protease inhibitor.
(17) Use of the probe set according to any one of (7) to(10) for therapy of hepatitis C based on a policy wherein responsiveness of a hepatitis C patient to a protease inhibitor is predicted and said protease inhibitor is administered

in cases where a protease inhibitor resistance mutation is not detected in said hepatitis C patient, while said protease inhibitor is not administered or administration of said protease inhibitor is ceased in cases where a protease inhibitor resistance mutation is detected in said hepatitis C patient.

(18) A method for detecting any of the following mutations a. to d. of NS3 protease in hepatitis C virus type 1b comprising carrying out fluorescent real-time PCR:

a. a mutation which results in replacement of Ala to Phe at position 156;
b. a mutation which results in replacement of Ala to Tyr at position 156;
c. a mutation which results in replacement of Val to Ile at position 158; and
d. a mutation which results in replacement of Val or Ile to Leu at position 132.

By carrying out real-time PCR using a probe set of the present invention, a mutation of interest can be identified simply, highly sensitively and specifically. By carrying out SNP analysis using a probe set of the present invention, susceptibility of an individual to a disease, effects and side effects of a drug, and the like can be simply investigated. Further, by identifying the mutant type of a virus using a probe set of the present invention, existence of a drug resistance virus or a pathogenic virus can be simply investigated.

BRIEF DESCRIPTION OF THE DRAWINGS

Fig. 1 shows diagrams showing the results of detection of the A156V mutation of the NS3 protease using a probe set of the present invention (halftone images). (A) shows the results obtained when A156V mutant-type NS3 protease cDNA was used as a template. The amounts of the template are $10^6$, 105, $10^4$, $10^3$ and $10^2$ copies from the left. (B) shows the results obtained when A156V mutant-type NS3 protease cDNA and an excess amount ($10^5$ copies) of wild-type NS3 protease cDNA were used as templates. The amounts of the template of A156V mutant-type NS3 protease cDNA are $10^6$, $10^5$, $10^4$ and $10^3$ copies from the left.

Fig. 2 shows diagrams showing the results of detection of the respective mutant types of the NS3 protease using a probe set of the present invention (halftone images). (A) A156T mutant type; (B) A156F mutant type; (C) A156V mutant type. The amounts of the template are $10^6$, $10^5$, $10^1$, $10^3$, $10^2$ and $10^1$ copies, respectively, from the left.

MODES FOR CARRYING OUT THE INVENTION

The probe set of the present invention is a nucleotide probe set to be used for specifying the type of the nucleotide at a nucleotide mutation site by fluorescent real-time PCR, which set comprises:

a detection probe composed of an oligonucleotide having: a nucleotide sequence comprising said mutation site in said target nucleic acid, said mutation site comprising a nucleotide of interest; or a nucleotide sequence complementary to said nucleotide sequence; said oligonucleotide having a fluorescent substance attached to the 5'-end and a quencher attached to the 3'-end; and

a counter probe composed of an oligonucleotide having: a nucleotide sequence comprising said mutation site, said mutation site comprising a nucleotide different from said nucleotide of interest; or a nucleotide sequence complementary to said nucleotide sequence, and

wherein said detection probe has a modification introduced such that the melting temperature thereof is not less than 3 °C higher than the melting temperature of the counter probe.

The mutation herein is preferably a nucleotide replacement.

<Detection Probe>

In the present invention, fluorescent real-time PCR means a method wherein an oligonucleotide probe labeled with a fluorescent substance at the 5'-end and with a quencher at the 3'-end is allowed to hybridize with a target template nucleic acid sequence and, when the complementary strand extends from a primer by the action of a thermostable DNA polymerase, the probe is degraded to emit fluorescence, whose intensity is then used to detect and quantify the sequence of interest (for example, US 6,214,979 B, US 5,804,375 B, US 5,487,972 B and US 5,210,015). That is, although the above probe specifically hybridizes with the template DNA in the annealing step, generation of fluorescence is usually suppressed even under irradiation of the excitation light since the quencher exists in the probe (FRET (fluorescence resonance energy transfer) phenomenon), but, in the subsequent extension reaction step, the probe that has hybridized

with the template is degraded by the 5'→3' exonuclease activity of the DNA polymerase, resulting in release of the fluorescent dye from the probe and cancellation of the suppression by the quencher, leading to emission of the fluorescence. Examples of such a probe include TaqMan probe (registered trademark).

In the detection probe contained in the probe set of the present invention, the labeling of the 5'- and 3'-ends may be carried out by using a fluorescent dye having a negative charge, such as a dye of the fluorescein family; a fluorescent dye having a neutral charge, such as a dye of the rhodamine family; or a fluorescent dye having a positive charge, such as a dye of the cyanine family. Examples of the dye of the fluorescein family include FAM, HEX, TET, JOE, NAN and ZOE. Examples of the dye of the rhodamine family include Texas Red, ROX, R110, R6G and TAMRA. FAM, HEX, TET, JOE, NAN, ZOE, ROX, R1 10, R6G and TAMRA are available from Perkin-Elmer (Foster City, Calif.), and Texas Red is commercially available from Molecular Probes, Inc. (Eugene, OR). Examples of the dye of the cyanine family include Cy2, Cy3, Cy5 and Cy7, which are commercially available from Amersham (Amersham Place, LittleChalfont, Buckinghamshire, England). Further, Iwoa, DABCYL, EDANS and the like may also be used.

A combination of a fluorescent substance and a quencher which may cause FRET can be appropriately selected among these substances and used. For example, FAM is most efficiently excited by a light having a wavelength of 488 nm and emits a light having a spectrum of 500 to 650 nm and an emission maximum of 525 nm. FAM is an appropriate donor label to be used together with, for example, TAMRA as a quencher, which has an excitation maximum of 514 nm.

Further, the combination of FAM and Iowa may also be used.

Further, examples of a nonfluorescent quencher which diffuses absorbed energy from a fluorescent dye include BlackHole Quenchers (registered trademark) commercially available from Biosearch Technologies, Inc. (Novato, Calif.).

The detection probe has a nucleotide sequence comprising a mutation site which comprises a nucleotide of interest, or a nucleotide sequence complementary thereto. The mutation of interest herein means the nucleotide to be detected, and means, for example, that, in cases where the type of the nucleotide at the mutation site is A or G and A is to be specifically detected, the nucleotide of the detection probe at this site is A (T, in the case of the complementary strand). The length of the detection probe is not restricted as long as it is a length with which the probe can specifically hybridize with the target sequence, and the probe preferably has a sequence of 15 to 18 nucleotides comprising the mutation site. The nucleotide of interest is preferably not located at an end of the probe.

The detection probe is modified such that the melting temperature (Tm) is not less than 3°C higher than that of the later-mentioned counter probe. In cases where plural counter probes are used, Tm of the detection probe is set to be not less than 3°C higher than that of the counter probe having the highest Tm. Such a modification is preferably a modification in the sugar-phosphate backbone, and examples thereof include modifications using the locked nucleic acid (LNA: registered trademark) and the peptide nucleic acid (PNA).

LNA herein means an RNA analog having a structure wherein, in the sugar-phosphate backbone, the oxygen atom at the 2'-position of ribose is methylene-crosslinked to the carbon atom at the 4'-position. By introducing a nucleic acid derivative comprising LNA instead of a normal nucleotide, upon hybridization with the target nucleic acid to form a double strand, stacking of the double strand is improved and the stability increases.

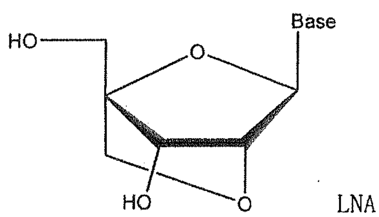

LNA

Further, the peptide nucleic acid means a structure as shown below.

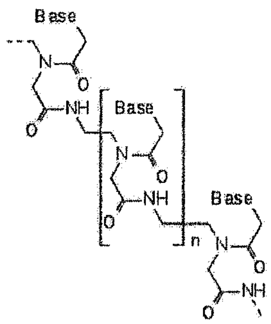

In order to modify the detection probe such that Tm is not less than 3 °C higher than that of the counter probe, a modifier such as LNA or PNA may be introduced based on the following equation.
The equation for Tm (unit is K; salt concentration is 1M NaCl) of the oligonucleotide according to the nearest-neighbor method is as follows: Tm (DNA/LNA) =ΔAH°/(AS°+R• ln[oligo]).
In this equation, R represents the gas constant (1.987 cal/K• mol), and [oligo] represents the molar concentration of the oligonucleotide.
As the nearest-neighbor parameters ΔH° and ΔS° in the case of a DNA oligonucleotide, the values described in Table 2 in SantaLucia, Proc. Natl. Acad. Sci. USA, 1998, Vol. 95, p1460-1465 are used.
As the nearest-neighbor parameters in the case of an oligonucleotide containing LNA, the values described in Table 4 in McTigue et al., Biochemistry, 2004, Vol. 43, p5388-5405 are used.
Tm of an oligonucleotide containing PNA is calculated according to the equation described in Giesen et al., Nucleic Acid Research, 1998, Vol. 26, No. 21, p5004-5006, which is as shown below: Tm (PNA) = 20.79+0.83• Tm (DNA)-26.13 $f_{pyr}$+0.44• L.
In this equation, $f_{pyr}$ represents the ratio of pyrimidine nucleotides, and L represents the nucleotide length of PNA.
In the presence of a magnesium ion and a monovalent cation, correction values of these Tm can be obtained according to Equation 4 in p. 5339 in Owczaryzy et al., Biochemistry, 2008, Vol. 47, p5336-5353. For example, the correction equation for the salt concentrations (150 mM $Na^+$, 1.5 mM $Mg^{2+}$, 0.3 mM dNTP) under the PCR conditions is as follows:

$$1/Tm(PCR) = 1/Tm(1M\ NaCl) + (4.29 \cdot f_{GC} - 3.95) \times 10^{-5} \cdot \ln[Na^+] + 9.40 \times 10^{-6} \cdot (\ln[Na^+])^2$$

In this equation, $f_{GC}$ represents the ratio of the purine nucleotides, and [$Na^+$] represents the molar concentration of the monovalent cation.
In cases where the detection probe has 15 to 18 nucleotides, it is preferred to introduce 2 to 5 modifiers.
In cases where the modification is carried out using LNA or PNA, the nucleotides to be modified are preferably nucleotides other than those located at the 5'-end and the 3'-end, and not located in succession. Further, the nucleotide of at least the mutation site is a modified nucleic acid such as LNA or PNA.
Since a difference of about 2°C exists in each article between the predicted value calculated by the nearest-neighbor method and the actual value, the predicted value of Tm of the detection probe is preferably sufficiently higher than that of the counter probe, but it is necessary to determine the value taking into consideration that, in cases where Tm is too high, it exceeds the upper limit of the practical temperature for PCR. Therefore, The predicted value of Tm is preferably 9 to 11°C higher than that of the counter probe; 70°C to 80°C is suitable as the predicted value; and the value is especially 70 to 76°C, more preferably 74 to 76°C.
The reaction temperature and the annealing temperature are preferably equivalent to the predicted value of Tm of the counter probe DNA (60 to 65°C), and Tm of the primer DNA is preferably between these (+5°C, 65-70°C).
The detection probe as described above is available from a custom synthesis service by Integrated DNA Technologies (Coralville, IA) or the like.

<Counter Probe>

The counter probe means a probe having: a nucleotide sequence comprising the mutation site which has a nucleotide different from the nucleotide of interest; or a nucleotide sequence complementary thereto; but having no modification with LNA, PNA or the like.

For example, in cases where adenine (A) is the nucleotide to be detected with the detection probe, the counter probe employed may be one or more types of oligonucleotides wherein the nucleotide is guanine (G), cytosine (C) or thymine (T). The counter probe may be one labeled with a fluorescent substance and a quencher at the 5'-and 3'-ends.
The length of the counter probe is not restricted as long as it allows specific hybridization with the target sequence, and the length is preferably 15 to 18 nucleotides.
By using the counter probe together with the detection probe, false detection due to nonspecific amplification can be prevented. The amount of the counter probe to be added is preferably the same with or larger than that of the detection probe.

<Primers>

As the primers, two types of primers, that is, a 5'-side primer (sense primer), which hybridizes with the 5'-side of the region in the target nucleic acid with which the probe hybridizes; and a 3'-side primer (antisense primer), which hybridizes with the 3'-side; are employed. One of these hybridizes with the sense strand of the target gene and the other hybridizes with the antisense strand, allowing amplification of the region between the both primers by PCR. Each primer is preferably set to a conservative region in the target nucleic acid. Further, the primers are preferably set to positions which allow amplification of a region having a length of 100 to 250 nucleotides.
The length of each primer is preferably 15 to 25 nucleotides, and Tm predicted based on the above-described equation for DNA oligonucleotide is practically lower than Tm of the detection probe and higher than the predicted value of Tm of the counter probe. More particularly, the predicted value of Tm is preferably 60 to 69°C, especially 65 to 69°C. A primer having the desired predicted value of Tm can be designed using software such as Primer Express (Applied Biosystems).

<Reaction Conditions>

Real-time PCR using the probes of the present invention can be carried out in a buffer containing the probe set, primers, target nucleic acid as a template, deoxyribonucleotide mixture (dNTPs) and thermostable DNA polymerase, under conditions for normal PCR.
In order to achieve the effect of the present invention, the annealing temperature in the PCR reaction is preferably 60 to 69°C, and preferably lower than Tm of the probe and the same as or higher than Tm of the counter probe. Usually, the extension reaction is carried out at a temperature higher than the annealing temperature, but the annealing and the extension reaction may be performed at the same temperature.
By repeating the temperature cycles of PCR sufficient for detection of the sequence of interest, and detecting, with a fluorescence detector, fluorescence due to the amplification, existence of the mutation of interest can be detected.
In the present specification, the term "thermostable DNA polymerase" means a polymerase which is stable under the reaction conditions for PCR and capable of catalyzing the reaction to polymerize deoxyribonucleotides to primers, and thereby extending the complementary strands of DNA, while hydrolyzing an annealed probe existing between the primers by its 5'→3' nuclease activity. As a representative thermostable DNA polymerase, a DNA polymerase isolated from *Thermus aquaticus* (Taq) is described in US 4,889,818 B, and a basic method to use it in PCR is described in Saiki et al., 1988, Science 239: 487-91.
In the present specification, "target nucleic acid" means a nucleic acid such as DNA or RNA which can be amplified by the PCR reaction and has one or more nucleotide mutation sites.
The target nucleic acid may be derived from human or a non-human mammal, bacterium, yeast, virus, viroid, mold, fungus, plant or another arbitrary organism; derived from arbitrary recombinants; or synthesized *in vitro* or by chemical synthesis.
Further, a sample containing the target nucleic acid can be used for the reaction.
The "sample" herein means a sample such as a tissue or body fluid isolated from an individual, and examples thereof include, but are not limited to, tissue biopsy materials, blood plasma, serum, whole blood, spinal fluid, lymph, sections of the outer skin, respiratory tract, intestinal tract, urogenital canal, tear, saliva, milk, blood cells, tumors and organs.
Further, a sample obtained from the soil, wastewater or the like may be used.
In cases where a mutation of a virus is to be detected, cDNA may be synthesized from the RNA genome of the virus using a reverse transcriptase, and the obtained cDNA or a product amplified from the cDNA may be used as the target nucleic acid.
In the present specification, the term "nucleotide mutation" means change in one or more nucleotides at a certain position(s) in a reference nucleotide sequence of a specific gene, and the "mutation" may be one which occurred either naturally or artificially. Single nucleotide polymorphisms (SNPs) are also included in the concept of the nucleotide mutation.
In cases where the nucleotide mutation is a mutation which is likely to cause a disease, susceptibility to the disease can

be predicted by identification of the nucleotide mutation by the method of the present invention. Further, in cases where the nucleotide mutation is a mutation involved in a side effect of a drug, the side effect of the drug can be predicted by identification of the nucleotide mutation by the method of the present invention.
In cases where the nucleotide mutation is a mutation specific to a species or a strain, the species or strain can be specified by identification of the nucleotide mutation by the method of the present invention. Further, in cases where the species or strain to be specified is a species or strain having pathogenicity, or a species or strain having drug resistance, detection of the pathogenic microbe or pathogenic virus, or detection of the drug-resistant strain can be carried out.
In cases where a mutant-type virus is to be detected, examples of the virus include human immunodeficiency virus (HIV), influenza virus, hepatitis C virus (HCV) and hepatitis B virus (HBV).
Examples of HCV include HCV type 1, and the present invention is especially suitably used for detection of a mutation in HCV-1b type.
At present, for HCV, drugs such as Telaprevir, which is a protease inhibitor, are being clinically developed, but it has been revealed that mutant-type viruses for which drugs are not effective exist, and therefore it is important to investigate the presence/absence of a mutation in the virus before drug administration and during the treatment period.
In addition to the above-described Telaprevir, protease inhibitors such as Boceprevir, Narlaprevir, Donaprevir (R7227/ITMN-191), MK-7009, TMC435, BMS65082, BI201335, MK-5172, GS9256 and ABT450 are also included in the protease inhibitor in the present invention as long as these achieve effects similar to that of the present invention.
For an HCV patient, investigation of the presence/absence of a mutation involved in drug resistance of HCV infected to the patient is very useful for decision of the medication policy.
As the mechanism of action of Telaprevir, a mechanism that inhibits the NS3 protease activity of HCV and thereby suppresses the growth of HCV has been proposed. However, in cases where a mutation has occurred in the region encoding the NS3 protease (SEQ ID NO:1), Telaprevir may become ineffective (Telaprevir resistance).

<Mutations at position 156>

Examples of such a mutation include mutations which replace the amino acid at position 156 (Ala in the wild type) of the NS3 protease (SEQ ID NO:2) with another amino acid such as Val, Thr, Ser, Phe or Tyr.
In the coding sequence of the wild-type NS3 protease, positions 466 to 468 of SEQ ID NO: is GCT, which encodes the amino acid Ala.
On the other hand, in A156V, the codon is changed to GTT due to a mutation at position 467 of SEQ ID NO: 1, and the encoded amino acid is changed to Val.
Examples of the detection probe for A156V are as shown below. (+) means a nucleotide wherein LNA is used for the sugar-phosphate backbone (same is also applied hereinafter). Although FAM is shown as an example of the fluorescent substance and Iowa is shown as an example of the quencher, other combinations may also be used.
5'(FAM)-G(+G)CA(+T)CT(+T)C(+C)GGG(+T)TG-3'(Iowa) (SEQ ID NO:6)
16 nucleotides, Tm 74.4°C (under the salt concentration for PCR)
Tm in the case where LNA is not introduced is 61.1 °C;
5'(FAM)-TC(+C)GGG(+T)TG(+C)TG(+T)GT-3'(Iowa) (SEQ ID NO:22)
15 nucleotides, Tm 74.4°C (under the salt concentration for PCR)
Tm in the case where LNA is not introduced is 61.9°C.
In A156F, the codon is changed to TTT due to mutations at positions 466 and 467 of SEQ ID NO:1, and the encoded amino acid is changed to Phe.
Examples of the detection probe for A156F are as follows.
5'(FAM)-G(+G)CA(+T)CT(+T)C(+C)GGT(+T)TGC-3'(Iowa) (SEQ ID NO:7)
17 nucleotides, Tm 74.8°C (under the salt concentration for PCR)
Tm in the case where LNA is not introduced is 62.3°C;
5'(FAM)-TC(+C)GG(+T)TTG(+C)TG(+T)ATGCA-3'(Iowa) (SEQ ID NO:23)
18 nucleotides, Tm 73.6°C (under the salt concentration for PCR)
Tm in the case where LNA is not introduced is 63.0°C.
In A156T, the codon is changed to ACT due to a mutation at position 466 of SEQ ID NO:1, and the encoded amino acid is changed to Thr.
Examples of the detection probe for A156T are as follows.
5'(FAM)-GGCA(+T)CT(+T)C(+C)GG(+A)CTGC-3'(Iowa) (SEQ ID NO:8)
17 nucleotides, Tm 74.1 °C (under the salt concentration for PCR)
Tm in the case where LNA is not introduced is 63.8°C;
5'(FAM)-TC(+C)GG(+A)CTG(+C)TG(+T)GTG-3'(Iowa) (SEQ ID NO:24)
18 nucleotides, Tm 73.9°C (under the salt concentration for PCR)
Tm in the case where LNA is not introduced is 62.4°C.

In A156S, the codon is changed to TCT due to a mutation at position 466 of SEQ ID NO:1, and the encoded amino acid is changed to Ser.
An example of the detection probe for A156S is as follows.
5'(FAM)-GGCA(+T)CT(+T)C(+C)GG(+T)CTGC-3'(Iowa) (SEQ ID NO:9)
17 nucleotides, Tm 74.4°C (under the salt concentration for PCR)
Tm in the case where LNA is not introduced is 63.8°C.
In A156Y, the codon is changed to TAT due to a mutation at position 466 of SEQ ID NO:1, and the encoded amino acid is changed to Tyr.
An example of the detection probe for A156Y is as follows.
5'(FAM)-TC(+C)GG(+T)ATG(+C)TG(+T)ATGCA-3'(Iowa) (SEQ ID NO:25)
18 nucleotides, Tm 73.2°C (under the salt concentration for PCR)
Tm in the case where LNA is not introduced is 61.9°C.
An example of the probe for detection of the wild type is as follows.
5'(FAM)-GCA(+T)CTTC(+C)GGG(+C)TGC-3'(Iowa) (SEQ ID NO:5)
16 nucleotides, Tm 73.7°C (under the salt concentration for PCR)
Tm in the case where LNA is not introduced is 64.4°C.
Therefore, in cases where a mutation which causes the amino acid replacement A156V is to be detected specifically, real-time PCR may be carried out using the above-described A156V probe as a detection probe and one or more types of other mutant-type probes and the wild type probe without modification with LNA as a counter probe(s). This also applies to the cases where other mutant types are to be detected specifically.

<Mutations at Position 155>

Examples of other mutations involved in Telaprevir resistance include mutations which replace the amino acid at position 155 (Arg in the wild type) of the NS3 protease (SEQ ID NO:2) with another amino acid such as Gly, Leu or Lys. The coding sequence of the wild-type NS3 protease has CGG at the positions corresponding to positions 463 to 465 of SEQ ID NO:1, which encodes the amino acid Arg.
On the other hand, in R155G, the codon is changed to GGG due to a mutation at position 463 of SEQ ID NO:1, and the encoded amino acid is changed to Gly.
An example of the detection probe for R155G is as follows.
5'(FAM)-TC(+G)GG(+G)CTG(+C)TG(+T)A(+T)G-3'(Iowa) (SEQ ID NO:11)
16 nucleotides, Tm 75.4°C (under the salt concentration for PCR)
Tm in the case where LNA is not introduced is 62.3°C.
In R155L, the codon is changed to CTG due to a mutation at position 464 of SEQ ID NO:1, and the encoded amino acid is changed to Leu.
An example of the detection probe for R155L is as follows.
5'(FAM)-TCC(+T)GG(+C)TG(+C)TG(+T)GTG-3'(Iowa) (SEQ ID NO:12)
16 nucleotides, Tm 74.3°C (under the salt concentration for PCR)
Tm in the case where LNA is not introduced is 62.9°C.
In R155K, the codon is changed to AAG due to mutations at positions 463 and 464 of SEQ ID NO:1, and the encoded amino acid is changed to Lys.
An example of the detection probe for R155K is as follows.
5'(FAM)-TCA(+A)GG(+C)TG(+C)TG(+T)ATGCA-3'(Iowa) (SEQ ID NO:13)
18 nucleotides, Tm 74.4°C (under the salt concentration for PCR)
Tm in the case where LNA is not introduced is 62.8°C.
An example of the probe for detection of the wild type is as follows.
5'(FAM)-TC(+C)GGG(+C)TG(+C)TG(+T)AT-3'(Iowa) (SEQ ID NO:10)
15 nucleotides, Tm 75.3°C (under the salt concentration for PCR)
Tm in the case where LNA is not introduced is 61.0°C.
Therefore, in cases where a mutation which causes the amino acid replacement R155G is to be detected specifically, real-time PCR may be carried out using the above-described R155G probe as a detection probe and one or more types of other mutant-type probes and the wild type probe without modification with LNA as a counter probe(s). This also applies to the cases where other mutant types are to be detected specifically.

<Combinations of Mutations at Position 156 and Position 158>

Further examples of the mutations include combinations of mutations at position 156 and position 158.
The wild type has Ala at position 156 and Val at position 158. In A156V/V158I, the codons are changed to GTT and

ATA, respectively, due to mutations at position 467 and position 472, and the encoded amino acids are changed to Val and Ile, respectively.
An example of the detection probe for A156V/V158I is as follows.
5'(FAM)-TC(+C)GGG(+T)TG(+C)T(+A)TA(+T)GCA-3'(Iowa) (SEQ ID NO:14)
18 nucleotides, Tm 74.3°C (under the salt concentration for PCR)
Tm in the case where LNA is not introduced is 62.0°C.

Further, in A156T/V158I, the codons are changed to ACT and ATA, respectively, due to mutations at position 466 and position 472, and the encoded amino acids are changed to Thr and Ile, respectively.
An example of the detection probe for A156V/V158I is as follows.
5'(FAM)-TC(+C)GG(+A)CTG(+C)T(+A)TA(+T)GCA-3'(Iowa) (SEQ ID NO:15)
18 nucleotides, Tm 73.6°C (under the salt concentration for PCR)
Tm in the case where LNA is not introduced is 61.3°C.

<Mutations at Position 54>

Examples of other mutations involved in Telaprevir resistance include mutations which replace the amino acid at position 54 (Thr in the wild type) of the NS3 protease (SEQ ID NO::2) with another amino acid such as Ala or Ser. The coding sequence of the wild-type NS3 protease has ACT at positions 160 to 162 of SEQ ID NO:1, which encodes the amino acid Thr.

On the other hand, in T54A, the codon is changed to GCT due to a mutation at position 160 of SEQ ID NO:1, and the encoded amino acid is changed to Ala.
An example of the detection probe for T54A is as follows.
5'(FAM)-G(+T)G(+T)GT(+T)GG(+G)CTG(+T)CT-3'(Iowa) (SEQ ID NO:17)
16 nucleotides, Tm 73.1 °C (under the salt concentration for PCR)
Tm in the case where LNA is not introduced is 60.2°C.

In T54S, the codon is changed to TCT due to a mutation at position 160 of SEQ ID NO: 1, and the encoded amino acid is changed to Ser.
An example of the detection probe for T54S is as follows.
5'(FAM)-CG(+T)G(+T)GT(+T)GG(+T)CTG(+T)CT-3'(Iowa) (SEQ ID NO:18)
17 nucleotides, Tm 72.3 °C (under the salt concentration for PCR)
Tm in the case where LNA is not introduced is 60.1°C.

An example of the probe for detection of the wild type is as follows.
5'(FAM)-CG(+T)G(+T)GT(+T)GG(+A)C'TG(+T)CT-3'(Iowa) (SEQ ID NO:16)
17 nucleotides, Tm 72.1 °C (under the salt concentration for PCR)
Tm in the case where LNA is not introduced is 60.1°C.

Therefore, in cases where a mutation which causes the amino acid replacement T54A is to be detected specifically, real-time PCR may be carried out using the above-described T54A probe as a detection probe and one or more types of other mutant-type probes including the wild type (without modification with LNA) as a counter probe(s).
This also applies to the cases where other mutant types are to be detected specifically.

<Mutations at Position 132>

Examples of other mutations involved in Telaprevir resistance include mutations which replace the amino acid at position 132 (Val or Ile in the wild type) of the NS3 protease (SEQ ID NO:2) with another amino acid such as Leu. The coding sequence of the wild-type NS3 protease has GTC or ATC at the positions corresponding to positions 394 to 396 of SEQ ID NO:1, which coding sequence encodes the amino acid Val or Ile.

In V/1132L, the codon is changed to CTC due to a mutation at position 394 of SEQ ID NO:1, and the encoded amino acid is changed to Leu.
An example of the detection probe for V/I132L is as follows.
5'(FAM)-CCAGGC(+C)T(+C)TCT(+C)CT(+A)C-3'(Iowa) (SEQ ID NO:21)
17 nucleotides, Tm 72.7 °C (under the salt concentration for PCR)
Tm in the case where LNA is not introduced is 61.2°C.

An example of the probe for detection of a wild type (V 132) is as follows.
5'(FAM)-CCAGGC(+C)TG(+T)CT(+C)CT(+A)C-3'(Iowa) (SEQ ID NO::19)
17 nucleotides, Tm 72.5 °C (under the salt concentration for PCR)
Tm in the case where LNA is not introduced is 61.8°C.

An example of the probe for detection of a wild type (I132) is as follows.
5'(FAM)-CCA(+G)GC(+C)TA(+T)CT(+C)CT(+A)C-3'(Iowa) (SEQ ID NO:20)

17 nucleotides, Tm 72.0 °C (under the salt concentration for PCR)
Tm in the case where LNA is not introduced is 58.4°C.

Therefore, in cases where a mutation which causes the amino acid replacement V/I132L is to be detected specifically, real-time PCR may be carried out using the above-described V/I132L probe as a detection probe and one or more types of other mutant-type probes including the wild type (without modification with LNA) as a counter probe(s). This also applies to the cases where other mutant types are to be detected specifically.

Among the above-described mutations, the following 4 types of virus mutants were newly discovered by the present inventors, and these may also be detected by fluorescent real-time PCR:

a. a mutation which results in replacement of Ala to Phe at position 156;
b. a mutation which results in replacement of Ala to Tyr at position 156;
c. a mutation which results in replacement of Val to Ile at position 158; and
d. a mutation which results in replacement of Val or Ile to Leu at position 132.

<Method for Predicting Response to Protease Inhibitor>

The present invention provides a method for predicting, using the above probe set, the response of a patient infected with HCV-1 b to a protease inhibitor. In one mode, the method comprises: providing a human patient-derived HCV-1b polynucleotide comprising a nucleotide sequence corresponding to position 156 of the HCV NS3 amino acid sequence; and determining whether or not a nucleotide(s) corresponding to Ala at position 156 in the amino acid sequence is/are mutated; wherein the existence of Ala at position 156 indicates continuous virological response (drug sensitivity) to a protease inhibitor.
In another mode, the method comprises: providing a human patient-derived HCV-1b polynucleotide comprising a nucleotide sequence corresponding to position 54 of the HCV NS3 amino acid sequence; and determining whether or not a nucleotide(s) corresponding to Thr at position 54 in the amino acid sequence is/are mutated; wherein the existence of Thr at position 54 indicates continuous virological response (sensitivity) to a protease inhibitor.
In another mode, the method comprises: providing a human patient-derived HCV-1b polynucleotide comprising a nucleotide sequence corresponding to position 132 of the HCV NS3 amino acid sequence; and determining whether or not a nucleotide(s) corresponding to Val or Ile at position 132 in the amino acid sequence is/are mutated; wherein the existence of Val or Ile at position 132 indicates continuous virological response (sensitivity) to a protease inhibitor.
In another mode, the method comprises: providing a human patient-derived HCV-1b polynucleotide comprising a nucleotide sequence corresponding to position 155 of the HCV NS3 amino acid sequence; and determining whether or not a nucleotide(s) corresponding to Arg at position 155 in the amino acid sequence is/are mutated; wherein the existence of Arg at position 155 indicates continuous virological response (sensitivity) to a protease inhibitor.
In another mode, the method comprises: providing a human patient-derived HCV-1b polynucleotide comprising a nucleotide sequence corresponding to position 156 and 158 of the HCV NS3 amino acid sequence; and determining whether or not nucleotides corresponding to Ala and Val at positions 156 and 158 in the amino acid sequence are mutated; wherein the existence of Ala and Val at positions 156 and 158 indicates continuous virological response (sensitivity) to a protease inhibitor.

Thus, the therapeutic policy for the human patient infected with HCV-1b can be determined. The method include, for example, a process wherein whether or not a nucleotide(s) corresponding to Ala at position 156 of the HCV NS3 amino acid sequence is/are mutated is determined, and, in cases where the sequence has Ala at position 156, protease inhibitor therapy is initiated, or, if the therapy has already been initiated, the therapy is continued; while in cases where the sequence is a mutant type, protease inhibitor therapy is not carried out, or, if the therapy has already been initiated, the therapy is ceased.
Further, the method include a process wherein whether or not a nucleotide(s) corresponding to Thr at position 54 of the HCV NS3 amino acid sequence is/are mutated is determined, and, in cases where the sequence has Thr at position 54, protease inhibitor therapy is initiated, or, if the therapy has already been initiated, the therapy is continued; while in cases where the sequence is a mutant type, protease inhibitor therapy is not carried out, or, if the therapy has already been initiated, the therapy is ceased.
Further, the method include a process wherein whether or not a nucleotide(s) corresponding to Val or Ile at position 132 of the HCV NS3 amino acid sequence is/are mutated is determined, and, in cases where the sequence has Val or Ile at position 132, protease inhibitor therapy is initiated, or, if the therapy has already been initiated, the therapy is continued; while in cases where the sequence is a mutant type, protease inhibitor therapy is not carried out, or, if the therapy has already been initiated, the therapy is ceased.
Further, the method include a process wherein whether or not a nucleotide(s) corresponding to Arg at position 155 of the HCV NS3 amino acid sequence is/are mutated is determined, and, in cases where the sequence has Arg at position 155, protease inhibitor therapy is initiated, or, if the therapy has already been initiated, the therapy is continued; while

in cases where the sequence is a mutant type, protease inhibitor therapy is not carried out, or, if the therapy has already been initiated, the therapy is ceased.
Further, the method include a process wherein whether or not nucleotides corresponding to Ala and Val at positions 156 and 158 of the HCV NS3 amino acid sequence are mutated is determined, and, in cases where the sequence has Ala and Val at positions 156 and 158, protease inhibitor therapy is initiated, or, if the therapy has already been initiated, the therapy is continued; while in cases where the sequence is a mutant type, protease inhibitor therapy is not carried out, or, if the therapy has already been initiated, the therapy is ceased.

<Diagnostic Kit for Predicting Responsiveness of Hepatitis C Patient to Protease Inhibitor>

The present invention provides a diagnostic kit for predicting responsiveness of a hepatitis C patient to a protease inhibitor, which kit comprises the above-described probe set. The diagnostic kit may comprise an instruction (package insert) wherein a therapeutic guideline is described, which therapeutic guideline explains that (i) a protease inhibitor may be administered in cases where a protease inhibitor resistance mutation is not detected; and (ii) administration of a protease inhibitor is ceased or not carried out in cases where a protease inhibitor resistance mutation is detected.

Examples of the present invention presented below are provided only for the illustration purpose, and do not limit the scope of the present invention. It is considered that many modes of the present invention included in the scope of the appended claims are evident to those skilled in the art by reference to the above text and the Examples below.

EXAMPLES

The amino acid at position 156 of the HCV-1b NS3 protease is Ala in the wild type, and it is replaced, as a result of nucleotide replacement, with Val, Phe, Thr, Ser or the like in a mutant-type virus. Here, real-time PCR was carried out using a fluorescent probe for specific detection of, among mutations leading to replacement of the amino acid at position 156, a mutation which causes replacement to Val at position 156, more particularly, the C→T replacement at position 467 of the nucleotide sequence encoding the NS3 protease.

<Primers>

In each of the upstream side and the downstream side of the nucleotides encoding the amino acid at position 156, a primer was set in a region which is common between the HCV NS3 protease regions of patients registered for clinical trial of Teraprevir and a public database (The Entrez Nucloetide Database) (positions 289 to 305 (Fw primer) and positions 487 to 503 (Re primer) of SEQ ID NO:1).
Fw primer 5'-TGCACCTGCGGCAGCTC-3' (SEQ ID NO:3)
Tm=69.2°C (under the salt concentration for PCR);
Re primer 5'-TCCACCGCCTTCGCRAC-3' (SEQ ID NO:4)
Tm=66.4168.4°C (under the salt concentration for PCR);
(R represents G or A).

<Detection Probe>

The detection probe was set in the region of 454 to 469 of SEQ ID NO:1, and FAM was bound to its 5'-end as a fluorescent dye, and Iowa was bound to its 3'-end as a quencher. For the 2nd, 5th, 8th, 10th and 14the nucleotide, LNA was used (using a custom synthesis service by Integrated DNA Technologies, Inc.). FAM Probe A156V 5'(FAM)-G(+G)CA(+T)CT(+T)C(+C)GGG(+T)TG-3'(Iowa) (SEQ ID NO:6); wherein for the nucleotides with (+), LNA was used for the sugar-phosphate backbone. Tm of the detection probe was as mentioned above.

<Counter Probes>

As counter probes, oligonucleotides corresponding to the wild type and the other mutations other than A156V were used. Tm of the counter probes were as mentioned above.
Probe WT
5'-GCATCTTCCGGGCTGC-3' (SEQ ID NO:5);
Probe A156F
5'-GGCATCTTCCGGTTTGC-3' (SEQ ID NO:7);
Probe A156T
5'-GGCATCTTCCGGACTGC-3' (SEQ ID NO:8);

Probe A156S
5'-GGCATCTTCCGGTCTGC-3' (SEQ ID NO:9).

<Quantitative PCR Reaction System>

As a template, a plasmid containing NS3 protease cDNA having the mutation A156V, with a copy number of $10^6$, $10^5$, $10^4$, $10^3$ or $10^2$, was used (Fig. 1A).
Using the reaction solution in Table 1, initial heating was performed at 50°C for 2 minutes and then at 95°C for 10 minutes, followed by 50 cycles of: 95°C for 15 seconds; and 65°C for 1 minute (2 steps). As a reaction device, PRISM 7900HT (Applied Biosystems) was used.
Further, in the coexistence of $10^5$ copies of a plasmid containing wild-type NS3 protease cDNA, the reaction was performed under the same conditions (Fig. 1B).
Table 1. Reaction system for detecting A156F

| Component | μL |
|---|---|
| template | 4 |
| DNase-free waster | 5.3 |
| 2x gene expression Mix | 10.0 |
| Fw primer (50 μ M) | 0.08 |
| Re primer (100 μ M) | 0.08 |
| FAM Probe A156 (50 μ M) | 0.1 |
| Probe A156T (50 μ M) | 0.1 |
| Probe A156S (50 μ M) | 0.1 |
| Probe A156V (50 μ M) | 0.1 |
| Probe WT (50 μ M) | 0.1 |
| Total | 20.0 |

From the results in Fig. 1A, it was revealed that the mutation A156V could be specifically detected.
Further, from the results in Fig 1B, it was shown that the mutant type can be detected even in cases where the mutant type exists at a ratio of only about 1% with respect to the wild type.

<Detection of Other Mutations>

Detection was also carried out for A156F and A156T.
The reaction was carried out in the same manner as described above using the following as a probe for detection of A156F and probes corresponding to the wild type and the other mutations as counter probes.
5'(FAM)-G(+G)CA(+T)CT(+T)C(+C)GGT(+T)TGC-3'(Iowa) (SEQ ID NO:7)
The reaction was carried out in the same manner as described above using the following as a probe for detection of A156T and probes corresponding to the wild type and the other mutations as counter probes.
5'(FAM)-GGCA(+T)CT(+T)C(+C)GG(+A)CTGC-3'(Iowa) (SEQ ID NO:8)
The results are shown in Fig. 2 together with the detection result for A156V. From these results, it was revealed that, by carrying out real-time PCR using a probe set of the present invention, a mutant type can be detected even in cases where the mutant type exists at a very small copy number (10 copies).

## Claims

**1.** A probe set to be used for identifying the type of the nucleotide at a mutation site of a target nucleic acid by fluorescent real-time PCR, said probe set comprising:

a detection probe composed of an oligonucleotide having: a nucleotide sequence comprising said mutation site in said target nucleic acid, said mutation site comprising a nucleotide of interest; or a nucleotide sequence complementary to said nucleotide sequence; said oligonucleotide having a fluorescent substance attached to the 5'-end and a quencher attached to the 3'-end; and
a counter probe composed of an oligonucleotide having: a nucleotide sequence comprising said mutation site,

said mutation site comprising a nucleotide different from said nucleotide of interest; or a nucleotide sequence complementary to said nucleotide sequence, and
wherein said detection probe has a modification introduced such that the melting temperature thereof is not less than 3 °C higher than the melting temperature of the counter probe.

**2.** The probe set according to claim 1, wherein said modification is modification by a locked nucleic acid.

**3.** The probe set according to claim 1 or 2, wherein said target nucleic acid is derived from a virus.

**4.** The probe set according to claim 3, wherein said virus is hepatitis C virus.

**5.** The probe set according to claim 4, wherein said hepatitis C virus belongs to type 1b.

**6.** The probe set according to claim 5, wherein said mutation is involved in drug resistance of said virus.

**7.** The probe set according to claim 6, wherein said drug is a protease inhibitor.

**8.** The probe set according to claim 6, wherein said drug is Telaprevir.

**9.** The probe set according to claim 7 or 8, wherein said protease is NS3 protease of hepatitis C virus.

**10.** The probe set according to claim 9, wherein said mutation is a mutation which results in replacement of any of the following amino acids in the wild-type NS3 protease:

(i) Ala at position 156;
(ii) Arg at position 155;
(iii) Ala at position 156 and Val at position 158;
(iv) Thr at position 54; and
(v) Val or Ile at position 132.

**11.** A method for identifying the type of the nucleotide at a mutation site of a target nucleic acid, comprising carrying out fluorescent real-time PCR by using the probe set according to any one of claims 1 to 10.

**12.** A method for predicting responsiveness of a hepatitis C patient to a protease inhibitor, comprising carrying out fluorescent real-time PCR by using the probe set according to any one of claims 7 to 10 to specify the type of the nucleotide at a mutation site involved in protease inhibitor resistance of hepatitis C virus type 1b and thereby determining whether or not said virus is a protease inhibitor-resistant virus.

**13.** A method for treating hepatitis C, comprising predicting the responsiveness of a hepatitis C patient to a protease inhibitor by the method according to claim 12, and said protease inhibitor is administered in cases where a protease inhibitor resistance mutation is not detected in said hepatitis C patient, while said protease inhibitor is not administered or administration of said protease inhibitor is ceased in cases where a protease inhibitor resistance mutation is detected in said hepatitis C patient.

**14.** A diagnostic kit for predicting responsiveness of a hepatitis C patient to a protease inhibitor, said kit comprising the probe set according to any one of claims 7 to 10.

**15.** The diagnostic kit according to claim 14, said diagnostic kit comprising an instruction wherein a therapeutic guideline is described, which therapeutic guideline explains that (i) a protease inhibitor may be administered in cases where a protease inhibitor resistance mutation is not detected; and (ii) administration of a protease inhibitor is ceased or not carried out in cases where a protease inhibitor resistance mutation is detected.

**16.** Use of the probe set according to any one of claims 7 to 10 for prediction of responsiveness of a hepatitis C patient to a protease inhibitor.

**17.** Use of the probe set according to any one of claims 7 to 10 for therapy of hepatitis C based on a policy wherein responsiveness of a hepatitis C patient to a protease inhibitor is predicted and said protease inhibitor is administered in cases where a protease inhibitor resistance mutation is not detected in said hepatitis C patient, while said protease

inhibitor is not administered or administration of said protease inhibitor is ceased in cases where a protease inhibitor resistance mutation is detected in said hepatitis C patient.

**18.** A method for detecting any of the following mutations a. to d. of NS3 protease in hepatitis C virus type 1b comprising carrying out fluorescent real-time PCR:

a. a mutation which results in replacement of Ala to Phe at position 156;
b. a mutation which results in replacement of Ala to Tyr at position 156;
c. a mutation which results in replacement of Val to Ile at position 158; and
d. a mutation which results in replacement of Val or Ile to Leu at position 132.

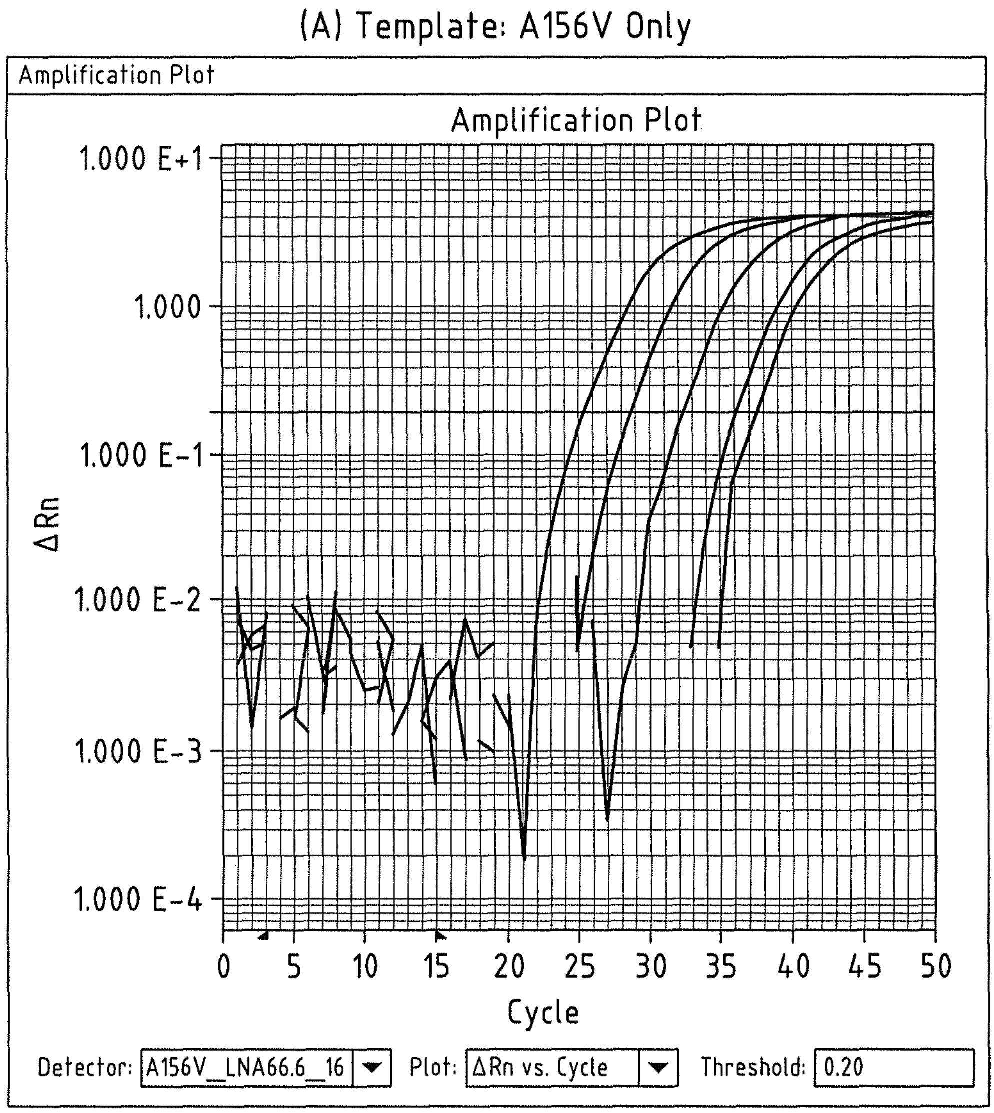
(A) Template: A156V Only
Amplification Plot
Amplification Plot
1.000 E+1
1.000
1.000 E-1
1.000 E-2
1.000 E-3
1.000 E-4
ΔRn
0
5
10
15
20
25
30
35
40
45
50
Cycle
Detector: A156V__LNA66.6__16
Plot: ΔRn vs. Cycle
Threshold: 0.20

Fig.1A

(B) Template: A156V in $10^5$ wild

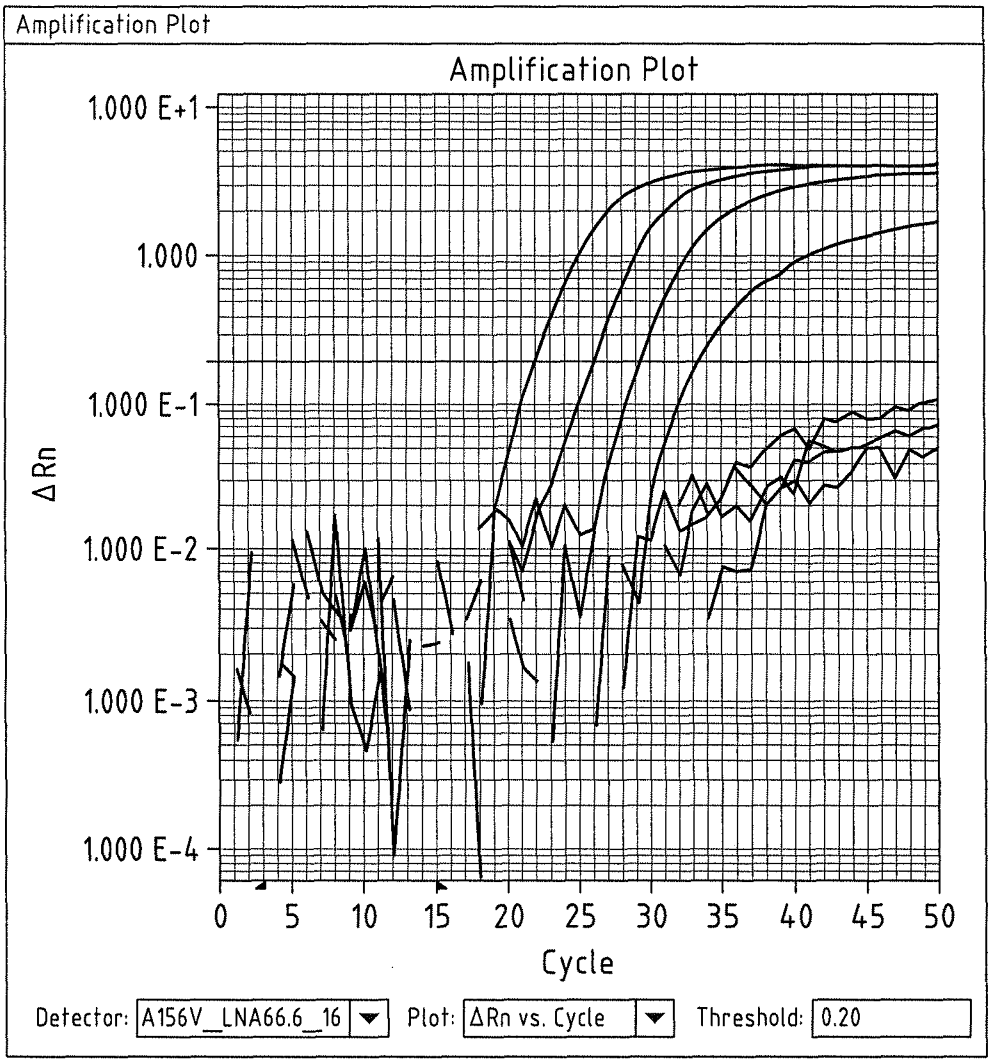


Fig.1B

(A) A156T

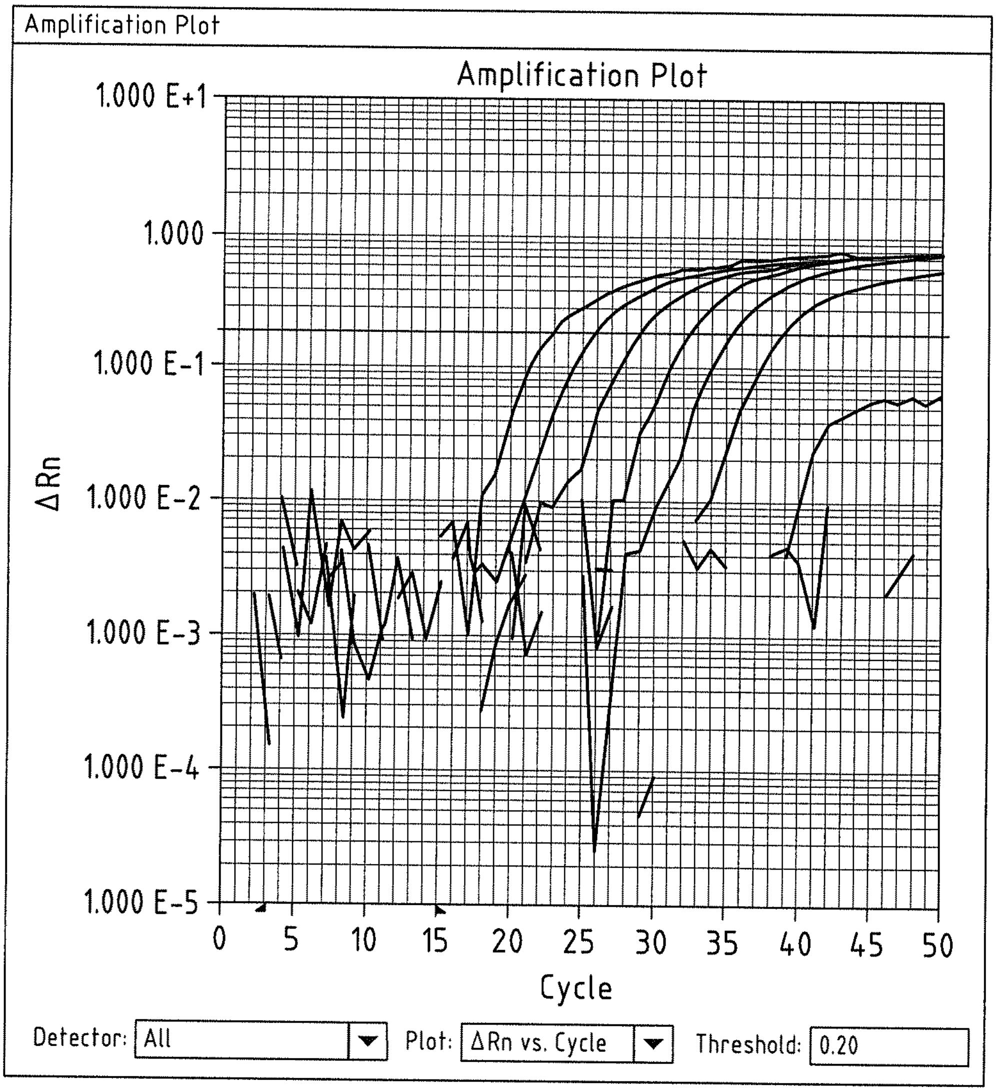


Fig.2A

(B) A156F

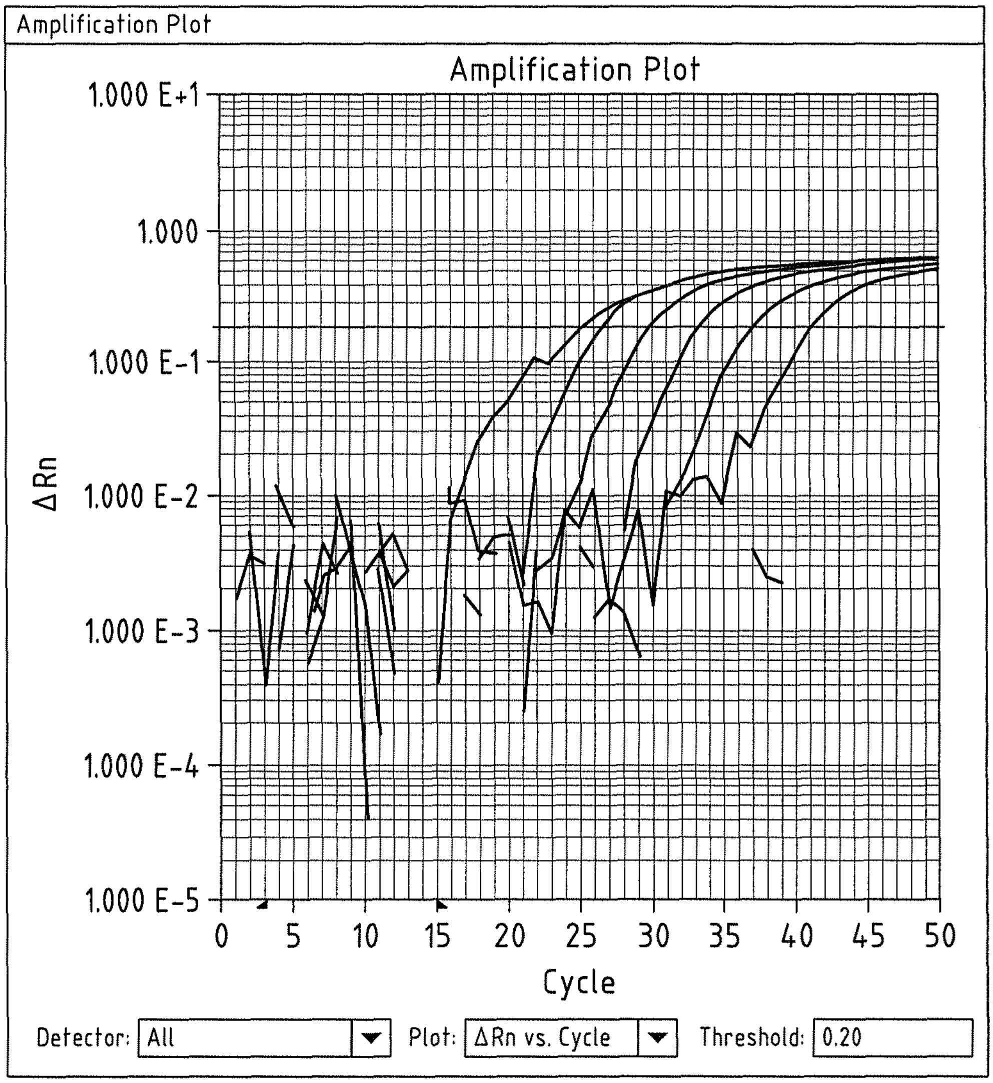


Fig.2B

(C) A156V

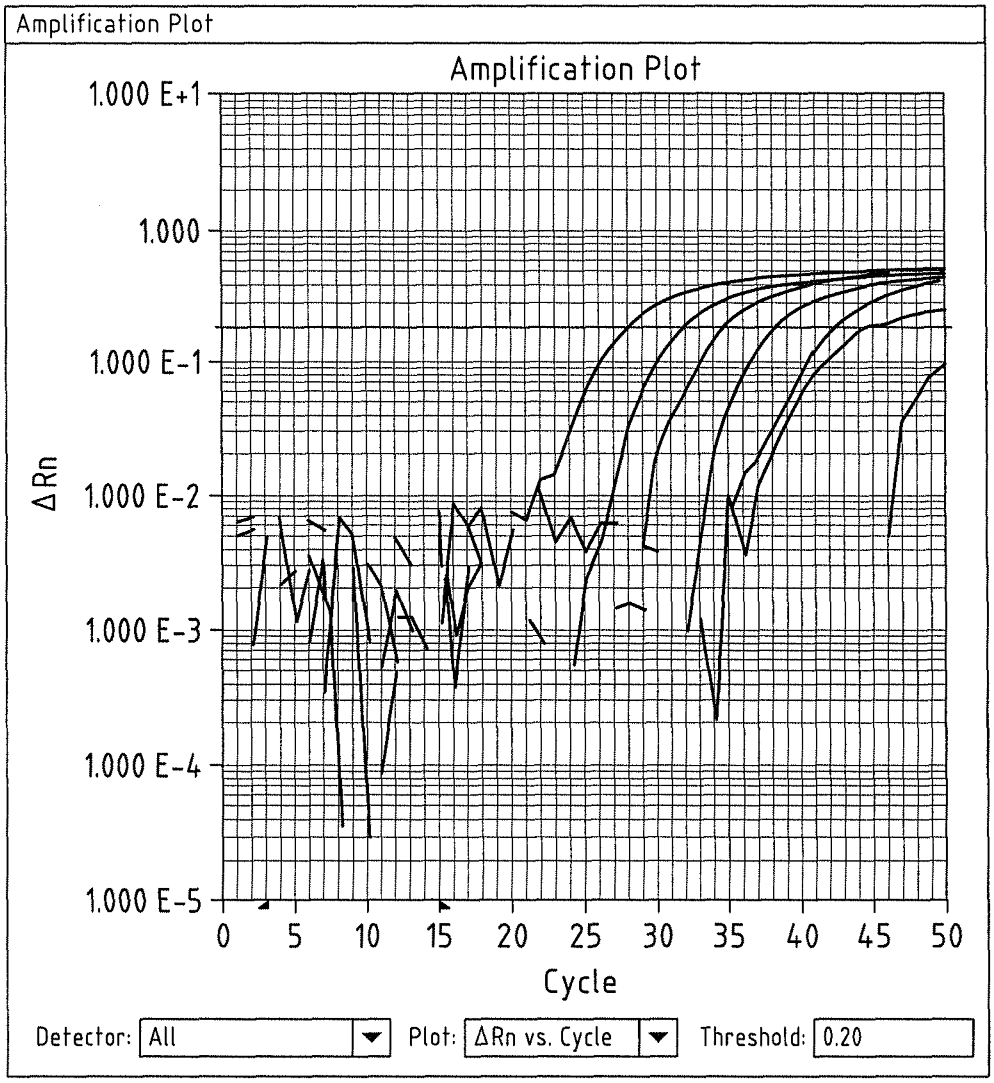


Fig.2C

**INTERNATIONAL SEARCH REPORT**

International application No.
PCT/JP2010/057135

A. CLASSIFICATION OF SUBJECT MATTER
*C12Q1/68*(2006.01)i, *C12N15/09*(2006.01)i

According to International Patent Classification (IPC) or to both national classification and IPC

B. FIELDS SEARCHED

Minimum documentation searched (classification system followed by classification symbols)
C12Q1/68, C12N15/09

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
CA/BIOSIS/MEDLINE/WPIDS(STN), JSTPlus/JMEDPlus/JST7580(JDreamII), PubMed

C. DOCUMENTS CONSIDERED TO BE RELEVANT

| Category* | Citation of document, with indication, where appropriate, of the relevant passages | Relevant to claim No. |
|---|---|---|
| X/Y | CHIEOCHANSIN, T. et al., Rapid detection of lamivudine-resistant hepatitis B virus mutations by PCR-based methods., Tohoku. J. Exp. Med., 2006, Vol.210, P.67-78 | 1-3,11/4-10, 12,14-16 |
| Y | CURRY, S. et al., Analysis of HCV resistance mutations during combination therapy with protease inhibitor boceprevir and PEG-IFN alpha-2b using TaqMan mismatch amplification mutation assay., J. Virol. Methods, 2008, Vol.153, P.156-162 | 4-10,12, 14-16 |
| Y | TONG, X. et al., Characterization of resistance mutations against HCV ketoamide protease inhibitors., Antiviral Res., 2008, Vol.77, P.177-185 | 4-10,12, 14-16 |

☒ Further documents are listed in the continuation of Box C.   ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 July, 2010 (12.07.10) | 20 July, 2010 (20.07.10) |
| Name and mailing address of the ISA/ Japanese Patent Office | Authorized officer |
| Facsimile No. | Telephone No. |

Form PCT/ISA/210 (second sheet) (July 2009)

**INTERNATIONAL SEARCH REPORT**

International application No.
PCT/JP2010/057135

C (Continuation). DOCUMENTS CONSIDERED TO BE RELEVANT

| Category* | Citation of document, with indication, where appropriate, of the relevant passages | Relevant to claim No. |
|---|---|---|
| A | MARKOVA, J. et al., A new allelic discrimination assay using locked nucleic acid-modified nucleotides (LNA) probes for detection of JAK2 V617F mutation., Leukemia and Lymphoma, 2007, Vol.48, No.3, P.636-639 | 1,2,11 |
| A | AMEZIANE, N. et al., Applicationof Locked Nucleic Acids and Real-Time PCR Methodology to Detection of the V249I Polymorphism of the CX3CR1 Chemokine Receptor, Preclinica, Vol.2, No.4, 2004, P.275-278 | 1,2,11 |
| A | KENNEDY, B. et al., Locked nucleic acids for optimizing displacement probes for quantitative real-time PCR., Anal. Biochem., 2006, Vol.348, P.294-299 | 1,2,11 |

Form PCT/ISA/210 (continuation of second sheet) (July 2009)

**INTERNATIONAL SEARCH REPORT**

International application No.
PCT/JP2010/057135

**Box No. II Observations where certain claims were found unsearchable (Continuation of item 2 of first sheet)**

This international search report has not been established in respect of certain claims under Article 17(2)(a) for the following reasons:

1. [X] Claims Nos.: 13, 17
because they relate to subject matter not required to be searched by this Authority, namely:
The above claims involve methods for treatment of the human body by therapy and thus relate to a subject matter on which this International Searching Authority is not required to carry out a search under the provisions of PCT Article 17(2)(a)(i) and PCT Rule 39.1(iv).

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet.

1. [ ] As all required additional search fees were timely paid by the applicant, this international search report covers all searchable claims.

2. [ ] As all searchable claims could be searched without effort justifying additional fees, this Authority did not invite payment of additional fees.

3. [ ] As only some of the required additional search fees were timely paid by the applicant, this international search report covers only those claims for which fees were paid, specifically claims Nos.:

4. [X] No required additional search fees were timely paid by the applicant. Consequently, this international search report is restricted to the invention first mentioned in the claims; it is covered by claims Nos.:
1-12, 14-16

**Remark on Protest**

[ ] The additional search fees were accompanied by the applicant's protest and, where applicable, the payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (July 2009)

**INTERNATIONAL SEARCH REPORT**

International application No.
PCT/JP2010/057135

Continuation of Box No.III of continuation of first sheet(2)

The invention according to claim 1 relates to "a probe set for use in the identification of the type of a nucleotide located in a mutation site on a target nucleic acid by means of a fluorescence real-time PCR, wherein the probe set comprises a detection probe and a counter probe, wherein the detection probe comprises an oligonucleotide which comprises a nucleotide sequence containing the mutation site on the target nucleic acid and also containing a nucleotide of interest in the mutation site or a nucleotide sequence complementary to the aforementioned nucleotide sequence, has a fluorescent substance added to the 5'-terminal and a quenching substance added to the 3'-terminal, and has, introduced therein, such a modification that the melting temperature of the probe becomes 3°C or more higher than that of the counter probe, and wherein the counter probe comprises an oligonucleotide which comprises a nucleotide sequence containing a mutation site and also containing a nucleotide that is different from the nucleotide of interest in the mutation site or a nucleotide sequence complementary to the aforementioned nucleotide sequence", i.e., a probe set for use in a fluorescence real-time PCR which comprises a detection probe and a counter probe.

On the other hand, the invention according to claim 18 relates to "a method for detecting any one mutation selected from the mutations "a" to "d" mentioned below in NS3 protease in type-C hepatitis virus 1b by means of a fluorescence real-time PCR:

a. a mutation of the substitution of Phe for Ala at position-156;
b. a mutation of the substitution of Tyr for Ala at position-156;
c. a mutation of the substitution of Ile for Val at position-158; and
d. a mutation of the substitution of Leu for Val or Ile at position-132", and there is found no statement about a probe in the invention according to claim 18.

Only a common matter between the invention according to claim 1 and the invention according to claim 18 is a matter that "a fluorescence real-time PCR" is employed. However, the matter is disclosed in, for example, the document shown below, and therefore cannot be regarded as a special technical feature in the meaning within PCT Rule 13.2, second sentence.

Consequently, it cannot be considered that the invention according to claim 1 and the invention according to claim 18 share any common special technical feature, and therefore these inventions cannot be regarded as being so linked as to form a single general inventive concept.

Document: J. Virol. Methods, 2008, Vol.153, P.156-162

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6214979 B
- US 5804375 B
- US 5487972 B
- US 5210015 A
- US 4889818 B


### Non-patent literature cited in the description


- *Nucleic Acid Research,* 2006, vol. 34 (8), e60
- *Journal of Virological Methods,* 2008, vol. 153, 156-162
- **SantaLucia.** *Proc. Natl. Acad. Sci. USA,* 1998, vol. 95, 1460-1465
- **McTigue et al.** *Biochemistry,* 2004, vol. 43, 5388-5405
- **Giesen et al.** *Nucleic Acid Research,* 1998, vol. 26 (21), 5004-5006
- **Owczaryzy et al.** *Biochemistry,* 2008, vol. 47, 5336-5353
- **Saiki et al.** *Science,* 1988, vol. 239, 487-91

SEQUENCE LISTING

<110> Mitsubishi Tanabe Pharma Corporation

<120> PROBE SET FOR IDENTIFICATION OF NUCLEOTIDE MUTATION, AND METHOD FOR IDENTIFICATION OF NUCLEOTIDE MUTATION

<130> A10008-10041

<150> JP2009-104468
<151> 2009-04-22

<160> 25

<170> PatentIn version 3.3

<210> 1
<211> 543
<212> DNA
<213> Hepatitis C virus

<220>
<221> CDS
<222> (1)..(543)

<400> 1
```
gcg cct atc acg gcc tat tcc caa cag acg cgg ggc cta ctc ggc tgt       48
Ala Pro Ile Thr Ala Tyr Ser Gln Gln Thr Arg Gly Leu Leu Gly Cys
1               5                   10                  15
atc atc act agc ctc aca ggt cgg gac aag aac cag gtc gag gga gag       96
Ile Ile Thr Ser Leu Thr Gly Arg Asp Lys Asn Gln Val Glu Gly Glu
            20                  25                  30
gtt cag gtg gtt tcc acc gca acg caa tct ttc ctg gcg acc tgt gtt      144
Val Gln Val Val Ser Thr Ala Thr Gln Ser Phe Leu Ala Thr Cys Val
        35                  40                  45
aac ggc gtg tgt tgg act gtc tac cat ggt gcc ggc tca aag acc cta      192
Asn Gly Val Cys Trp Thr Val Tyr His Gly Ala Gly Ser Lys Thr Leu
    50                  55                  60
gcc ggc cca aag ggc cca atc acc caa atg tac acc aat gtg gac cag      240
Ala Gly Pro Lys Gly Pro Ile Thr Gln Met Tyr Thr Asn Val Asp Gln
65                  70                  75                  80
gac ctc gtc ggc tgg cag gcg ccc ccc ggg tcg cgc tcc ttg aca cca      288
Asp Leu Val Gly Trp Gln Ala Pro Pro Gly Ser Arg Ser Leu Thr Pro
                85                  90                  95
tgc acc tgc ggc agc tcg gac ctt tat ttg gtc acg cgg cat gcc gat      336
Cys Thr Cys Gly Ser Ser Asp Leu Tyr Leu Val Thr Arg His Ala Asp
            100                 105                 110
gtc att ccg gtg cgc cgg cgg ggc gac aac agg ggg agc ctg ctc tct      384
Val Ile Pro Val Arg Arg Arg Gly Asp Asn Arg Gly Ser Leu Leu Ser
        115                 120                 125
ccc agg cct atc tcc tac ttg aag ggt tct tcg ggt ggg cca ctg ctc      432
Pro Arg Pro Ile Ser Tyr Leu Lys Gly Ser Ser Gly Gly Pro Leu Leu
    130                 135                 140
tgc cct ttg ggg cac gtt gtg ggc atc ttc cgg gct gct gta tgc acc      480
Cys Pro Leu Gly His Val Val Gly Ile Phe Arg Ala Ala Val Cys Thr
145                 150                 155                 160
cgg ggg gtt gcg aag gcg gtg gac ttt ata ccc gtt gag tct atg gaa      528
Arg Gly Val Ala Lys Ala Val Asp Phe Ile Pro Val Glu Ser Met Glu
                165                 170                 175
act act atg cgg tct                                                  543
Thr Thr Met Arg Ser
            180
```

<210> 2
<211> 181
<212> PRT
<213> Hepatitis C virus

<400> 2

```
Ala Pro Ile Thr Ala Tyr Ser Gln Gln Thr Arg Gly Leu Leu Gly Cys
1               5                   10                  15
Ile Ile Thr Ser Leu Thr Gly Arg Asp Lys Asn Gln Val Glu Gly Glu
            20                  25                  30
Val Gln Val Val Ser Thr Ala Thr Gln Ser Phe Leu Ala Thr Cys Val
        35                  40                  45
Asn Gly Val Cys Trp Thr Val Tyr His Gly Ala Gly Ser Lys Thr Leu
    50                  55                  60
Ala Gly Pro Lys Gly Pro Ile Thr Gln Met Tyr Thr Asn Val Asp Gln
65                  70                  75                  80
Asp Leu Val Gly Trp Gln Ala Pro Pro Gly Ser Arg Ser Leu Thr Pro
                85                  90                  95
Cys Thr Cys Gly Ser Ser Asp Leu Tyr Leu Val Thr Arg His Ala Asp
            100                 105                 110
Val Ile Pro Val Arg Arg Arg Gly Asp Asn Arg Gly Ser Leu Leu Ser
        115                 120                 125
Pro Arg Pro Ile Ser Tyr Leu Lys Gly Ser Ser Gly Gly Pro Leu Leu
    130                 135                 140
Cys Pro Leu Gly His Val Val Gly Ile Phe Arg Ala Ala Val Cys Thr
145                 150                 155                 160
Arg Gly Val Ala Lys Ala Val Asp Phe Ile Pro Val Glu Ser Met Glu
                165                 170                 175
Thr Thr Met Arg Ser
            180
```

<210> 3
<211> 17
<212> DNA
<213> Artificial sequence

<220>
<223> 5′primer for 156

<400> 3
tgcacctgcg gcagctc 17

<210> 4
<211> 17
<212> DNA
<213> Artificial sequence

<220>
<223> 3′primer for 156

<400> 4
tccaccgcct tcgcrac 17

<210> 5
<211> 16
<212> DNA
<213> Artificial sequence

<220>
<223> probe for 156wild

<400> 5
gcatcttccg ggctgc 16

<210> 6
<211> 16
<212> DNA
<213> Artificial sequence

<220>
<223> probe for 156V

<400> 6
ggcatcttcc gggttg 16

<210> 7
<211> 17
<212> DNA
<213> Artificial sequence

<220>
<223> probe for 156F

<400> 7
ggcatcttcc ggtttgc 17

<210> 8
<211> 17
<212> DNA
<213> Artificial sequence

<220>
<223> probe for 156T

<400> 8
ggcatcttcc ggactgc 17

<210> 9
<211> 17
<212> DNA
<213> Artificial sequence

<220>
<223> probe for 156S

<400> 9
ggcatcttcc ggtctgc 17

<210> 10
<211> 15
<212> DNA
<213> Artificial sequence

<220>
<223> probe for 155wild

<400> 10
tccgggctgc tgtat 15

<210> 11

<211>  16
<212>  DNA
<213>  Artificial sequence

<220>
<223>  probe for 155G

<400>  11
tcggggctgc tgtatg                                                                    16

<210>  12
<211>  16
<212>  DNA
<213>  Artificial sequence

<220>
<223>  probe for 155L

<400>  12
tcctggctgc tgtgtg                                                                    16

<210>  13
<211>  18
<212>  DNA
<213>  Artificial sequence

<220>
<223>  probe for 155K

<400>  13
tcaaggctgc tgtatgca                                                                  18

<210>  14
<211>  18
<212>  DNA
<213>  Artificial sequence

<220>
<223>  probe for 156V158I

<400>  14
tccgggttgc tatatgca                                                                  18

<210>  15
<211>  18
<212>  DNA
<213>  Artificial sequence

<220>
<223>  probe for 156T158I

<400>  15
tccggactgc tatatgca                                                                  18

<210>  16
<211>  17
<212>  DNA
<213>  Artificial sequence

<220>

<223> probe for 54wild

<400> 16
cgtgtgttgg actgtct 17

<210> 17
<211> 16
<212> DNA
<213> Artificial sequence

<220>
<223> probe for 54A

<400> 17
gtgtgttggg ctgtct 16

<210> 18
<211> 17
<212> DNA
<213> Artificial sequence

<220>
<223> probe for 54S

<400> 18
cgtgtgttgg tctgtct 17

<210> 19
<211> 17
<212> DNA
<213> Artificial sequence

<220>
<223> probe for 132wild(V)

<400> 19
ccaggcctgt ctcctac 17

<210> 20
<211> 17
<212> DNA
<213> Artificial sequence

<220>
<223> probe for 132wild(I)

<400> 20
ccaggcctat ctcctac 17

<210> 21
<211> 16
<212> DNA
<213> Artificial sequence

<220>
<223> probe for 132L

<400> 21
ccaggccctc tcctac 16

```
<210>  22
<211>  15
<212>  DNA
<213>  Artificial sequence

<220>
<223>  probe for 156V

<400>  22
tccgggttgc tgtgt                                                     15


<210>  23
<211>  18
<212>  DNA
<213>  Artificial sequence

<220>
<223>  probe for 156F

<400>  23
tccggtttgc tgtatgca                                                  18


<210>  24
<211>  16
<212>  DNA
<213>  Artificial sequence

<220>
<223>  probe for 156T

<400>  24
tccggactgc tgtgtg                                                    16


<210>  25
<211>  18
<212>  DNA
<213>  Artificial sequence

<220>
<223>  probe for 156Y

<400>  25
tccggtatgc tgtatgca                                                  18
```